# EUROPEAN PATENT SPECIFICATION

(11) **EP 0 873 293 B1**
(45) Date of publication and mention of the grant of the patent: **25.07.2001**
(21) Application number: 96942912.5
(22) Date of filing: 10.12.1996
(51) Int. Cl.: C07C 45/59, C07C 47/277, C07C 45/60

(54) **IMPROVED PROCESS FOR THE PREPARATION OF ALKYL OR ARYL ALDEHYDE INTERMEDIATES**
VERFAHREN ZUR HERSTELLUNG VON ALKYL ODER ARYL ALDEHYDZWISCHENPRODUKTEN
PROCEDE AMELIORE DE PREPARATION D'INTERMEDIAIRES ALDEHYDES D'ALKYLE OU D'ARYLE

(30) Priority: 22.12.1995 US 577581
(43) Date of publication of application: 28.10.1998
(73) Proprietor: Abbott Laboratories, Abbott Park, Illinois 60064-3500 (US)
(72) Inventor: MONTE, William, T., Lincolnshire, IL 60069 (US)
(74) Representative: Modiano, Guido, Dr.-Ing.
(86) International application number: US9619432
(87) International publication number: WO9723439

(56) References cited:
- EP-A- 0 329 464
- J. ORG. CHEM. (JOCEAH,00223263);83; VOL.48 (10); PP.1767-9, PFIZER INC.;CENT. RES.; GROTON; 06340; CT; USA (US), XP002025437 VOLKMANN R A ET AL: "Synthesis of functionalized aliphatic aldehydes via a copper-catalyzed Grignard coupling reaction"

## Description

The present invention pertains to the preparation of alkyl or aryl aldehydes using Grignard protected aldehydes and alkyl or benzyl halides. The formed aldehydes are useful as intermediates in the synthesis of pharmaceutical and agricultural products.

### Background of the Invention

Amine oxalates are widely recognized as useful intermediates in the preparation of pharmaceutically active compounds. By way of example, arbutamine oxalate is the penultimate intermediate precursor in the formation of arbutamine hydrochloride. Typically, the amine oxalate is formed by reductive amination of an aldehyde and the oxalate salt of an amine compound such as epinephrine or norepinephrine. The formation of arbutamine oxalate, for example, proceeds by reductively aminating (R)-norepinephrine with 4-(4-benzyloxyphenyl) butanal. It can thus be seen that preparation of the aldehyde is an important step in the overall synthetic scheme.

Existing methods for preparation of such aldehydes require numerous oxidation and reduction steps (see e.g., Tamura et al., *J. Synthesis,* June 1971, 303-305; Tanis et al., *Tet. Lett.,* 23:3115-3118, 1982).

R. A. Volkmann et al., Journal of Organic Chemistry, volume 48, pages 1767-1769 (1983) disclose a process for making a coupled product by reacting a Grignard reagent of a protected aldehyde with an alkyl halide. The Grignard reagent is added to a mixture of the alkyl halide and a copper catalyst in tetrahydrofuran.

There continues to be a need in the art for a simple and economic means for producing aldehydes for use in synthesizing pharmaceutical compounds.

### Brief Summary of the Invention

The present invention provides an improved process for the preparation of alkyl or aryl aldehydes, which aldehydes are intermediates in the synthesis of pharmaceutical and agricultural products. The process of preparing a coupled product according to the invention comprises coupling a Grignard protected aldehyde to a halide in the presence of a copper catalyst to form a coupled product, characterized in that the halide and copper catalyst are combined and then added to the Grignard protected aldehyde. In particular, in one embodiment of the improved process, a Grignard protected aldehyde magnesium copper halide is reacted with an alkyl or aryl halide to form a coupled product. In addition, the coupled product may be selectively deprotected by hydrolysis.

A process of the present invention, when compared to existing methods, reduces the overall number of synthetic steps needed to prepare the aldehyde, increases the yield of aldehyde, and increases the purity of aldehyde. These advantages allow for significant savings of time and money when preparing such aldehydes.

### Detailed Description of the Invention

To prepare an alkyl aldehyde, a Grignard protected aldehyde is coupled to an alkyl halide in the presence of a copper catalyst to form a coupled product. The Grignard protected aldehyde has the formula R-MgX, where R represents any suitable compound and X is a halide. A preferred R is a cyclic compound of 5 or 6 atoms wherein two of the ring atoms are oxygen. Especially preferred R groups are dioxolane and dioxane. A preferred halide is bromide.

The Grignard protected aldehyde is prepared using a Grignard reagent of the formula MgX, where X is defined above. Formation of the Grignard protected aldehyde is preferably performed in tetrahydrofuran (THF) at a temperature of from about 15°C to about 35°C and, more preferably from about 20°C to about 28°C to avoid decomposition. The need to filter off excess magnesium from the reaction mixture can be accomplished by using 1 equivalent of magnesium per equivalent of starting compound.

The Grignard protected aldehyde is then reacted with an alkyl halide in the presence of a copper catalyst to provide a coupled product. A preferred copper catalyst is an alkali metal copper halide such as Li₂CuCl₄. The alkyl halide has the formula R'X', where R' is alkyl and X' is halide.

The present invention relates to a method of adding the reactants which results in greater yields of coupled product. The alkyl halide and copper catalyst are combined and then added to the Grignard protected aldehyde to yield coupled product. This order of combining reagents results in the greatest yield of coupled product. Byproduct formation can be minimized by using about 1.4 to 1.6 equivalents of the Grignard protected aldehyde per equivalent of the alkyl halide.

The reaction of the Grignard protected aldehyde with the alkyl halide is performed at a cool temperature typically in the range of -5°C to 0°C. The coupling reaction is only mildly exothermic and can be maintained at those cool temperatures for about 3 to 5 hours. The coupling reaction mixture is then allowed to warm to about room temperature over the next 15 to 20 hours.

The coupled product formed by the above coupling reaction may also be hydrolytically deprotected *in situ.* Prior to hydrolysis, the coupled product is extracted from the coupling reaction mixture. Typically, extraction is accomplished by quenching the coupling reaction with ammonium chloride, extracting with ethyl acetate, washing the organic layer with water, drying the extract (MgSO₄), filtering off byproducts and concentrating to form an organic residue, which is then hydrolyzed.

Hydrolytic deprotection is accomplished using acid hydrolysis with weak organic and inorganic acids. One example of such a weak acid includes, but is not intended to be limited to, formic acid. Preferably, the formic acid is an aqueous formic acid solution of from about 70% to about 90% and, more preferably about 80% formic acid. Acid hydrolysis is preferably performed at room temperature.

Following acid hydrolysis, the formed aldehyde is collected using standard procedures well known in the art. Typically, the hydrolysis solution is diluted with a salt solution such as, but not intended to be limited to, sodium chlorides solutions such as brine and extracted with methylene chloride. Residual acid is removed by washing the organic extract with a base such as bicarbonate. The washed organic material is dried (MgSO₄) and concentrated (e.g., vacuum distillation). The concentrated material is then dissolved in alcohol (e.g., isopropanol), treated with a bisulfite (e.g., sodium bisulfite) and heated to about 60°C to 70°C. Upon cooling, the bisulfite adduct of the aldehyde product crystallizes out and can be collected and dried.

Preparation of an aryl aldehyde is accomplished in a similar manner to the above procedures except that the Grignard protected aldehyde is reacted with a benzyl halide instead of an alkyl halide. In a preferred embodiment, an aryl aldehyde is a phenyl aldehyde. Preferred embodiments of reaction conditions are the same as set forth above. A detailed description of the preparation of 4-(4-benzyloxyphenyl)butanal using a process of the present invention is set forth in detail below in the Example. That Example is illustrative of certain preferred embodiments and is not limiting of the specification and claims in any way.

### Example 1: Preparation of 4-(4-benzyloxybenzyl)butanal

A Grignard solution is prepared by adding 10.6 mL (16.3g, 90.2 mmol) of 2-(2-bromoethyl)-1,3-dioxolane in 80 mL of THF to 2.4 g (99.26 mmol) of magnesium turnings. The addition is carried out so as to maintain a reaction temperature below 28°C. After the addition is complete, the reaction mixture is stirred at 20-25°C for 1 hour. The Grignard solution is then cooled to 0 to -5°C and benzyloxybenzyl chloride in 80 mL of THF and 3 mL of 0.1 M Li₃CuCl₄ in THF is added. The reaction is stirred for 5 hours at -5°C to 0°C and allowed to warm to room temperature over 17 hours. The reaction is quenched with 200 mL of 1N aqueous ammonium chloride and extracted into ethyl acetate. The aqueous layers are back extracted with ethyl acetate and the pooled organics are dried over MgSO₄. The solvent is removed by rotary evaporation under vacuum and the residue is re-dissolved in ether and filtered. The filtrate is concentrated by rotary evaporation under vacuum to give 17.0 g of crude product as a yellow liquid.

The crude 2-[2-(4-benzyloxyphenyl) ethyl]-1,3-dioxolane is mixed with 40 mL of 80% aqueous formic acid and stirred at room temperature for 1 hour. The reaction is then diluted in 100 mL of brine and extracted into 100 mL of methylene chloride. The aqueous solution is back extracted with methylene chloride and the pooled organics are washed with aqueous saturated bicarbonate solution. The organics are dried over MgSO₄ and concentrated by rotary evaporation under vacuum. The residue (16 g) is dissolved in 20 mL of isopropyl alcohol and is added to a mixture of 16 g sodium bisulfite in 50 mL of water warmed to 70°C. The hot chloride is added. The resulting precipitate is collected and washed with water, isopropanol, and methylene chloride. The crude bisulfite adduct is slurried in about 60 mL of methylene chloride and collected by filtration. After drying 12.6 g of desired bisulfite product, a white solid is obtained.

The solid bisulfite product is vigorously stirred in a mixture of 100 mL of 1N aqueous sodium hydroxide and 100 mL of isopropyl ether. After 2 hours, all the solids are dissolved and the organics are separated and washed with brine. The organics are dried over MgSO₄ and concentrated by rotary evaporation under vacuum to yield 8.7 g (53%) of the desired aldehyde as a colorless liquid.

## Claims

1. A process of preparing a coupled product comprising coupling a Grignard protected aldehyde to a halide in the presence of a copper catalyst to form a coupled product, characterized in that the halide and copper catalyst are combined and then added to the Grignard protected aldehyde.

2. A process in accordance with claim 1 further comprising hydrolytically deprotecting the coupled product to form the aldehyde.

3. The process of claim 1 wherein the halide is an alkyl halide or a benzyl halide.

4. The process of claim 1 wherein the Grignard protected aldehyde has the formula R-MgX, where R is a cyclic compound of 5 or 6 atoms that contains two oxygen atoms and X is halide.

5. The process of claim 4 wherein the cyclic compound is dioxane or dioxolane.

6. The process of claim 4 wherein X is bromide.

7. The process of claim 1 wherein the copper catalyst is an alkali metal copper halide.

8. The process of claim 7 wherein the copper catalyst is Li₂CuCl₄.

9. The process of claim 2 wherein the coupled product is hydrolytically deprotected by acid hydrolysis.

10. The process of claim 9 wherein the acid hydrolysis is accomplished with weak organic and inorganic acids.

11. The process of claim 10 wherein acid hydrolysis is accomplished by adding aqueous formic acid to the coupled product.

12. The process of claim 3 wherein the benzyl halide is benzyloxybenzyl chloride.

## Patentansprüche

1. Ein Verfahren zur Herstellung eines verknüpften Produktes, das die Anknüpfung eines Grignard-geschützten Aldehyds an ein Halid in Anwesenheit von einem Kupferkatalysator umfaßt, um ein verknüpftes Produkt zu bilden, dadurch gekennzeichnet, daß das Halid und der Kupferkatalysator vereinigt werden und dann zu dem Grignard-geschützten Aldehyd hinzugefügt werden.

2. Ein Verfahren in Übereinstimmung mit Anspruch 1, das weiter die hydrolytische Deprotektion des verknüpften Produkts umfaßt, um das Aldehyd zu bilden.

3. Das Verfahren gemäß Anspruch 1, worin das Halid ein Alkylhalid oder ein Benzylhalid ist.

4. Das Verfahren gemäß Anspruch 1, worin das Grignardgeschützte Aldehyd die Formel R-MgX hat, worin R eine zyklische Verbindung von 5 oder 6 Atomen ist, die zwei Sauerstoffatome enthält und worin X ein Halid ist.

5. Das Verfahren gemäß Anspruch 4, worin die zyklische Verbindung Dioxan oder Dioxolan ist.

6. Das Verfahren gemäß Anspruch 4, worin X Bromid ist.

7. Das Verfahren gemäß Anspruch 1, worin der Kupferkatalysator ein Alkalimetallkupferhalid ist.

8. Das Verfahren gemäß Anspruch 7, worin der Kupferkatalysator Li₂CuCl₄ ist.

9. Das Verfahren gemäß Anspruch 2, worin das verknüpfte Produkt hydrolytisch durch Säurehydrolyse deprotektiert wird.

10. Das Verfahren gemäß Anspruch 9, worin die Säurehydrolyse mit schwachen organischen und anorganischen Säuren erreicht wird.

11. Das Verfahren gemäß Anspruch 10, worin die Säurehydrolyse durch Zugabe von wässeriger Ameisensäure zu dem verknüpften Produkt erreicht wird.

12. Das Verfahren gemäß Anspruch 3, worin das Benzylhalid Benzyloxybenzylchlorid ist.

## Revendications

1. Procédé de préparation d'un produit couplé comprenant le couplage d'un aldéhyde protégé selon Grignard à un halogénure en présence d'un catalyseur au cuivre pour former un produit couplé, caractérisé en ce que l'halogénure et le catalyseur au cuivre sont combinés et ensuite ajoutés à l'aldéhyde protégé selon Grignard.

2. Procédé selon la revendication 1 comprenant en outre la dé-protection par hydrolyse du produit couplé pour former l'aldéhyde.

3. Procédé selon la revendication 1, caractérisé en ce que l'halogénure est un halogénure d'alkyle ou un halogénure de benzyle.

4. Procédé selon la revendication 1, caractérisé en ce que l'aldéhyde protégé selon Grignard a la formule R-MgX, où R est un composé cyclique de 5 ou 6 atomes qui contient deux atomes d'oxygène et X est un halogénure.

5. Procédé selon la revendication 4, caractérisé en ce que le composé cyclique est le dioxane ou le dioxolane.

6. Procédé selon la revendication 4, caractérisé en ce que X est le bromure.

7. Procédé selon la revendication 1, caractérisé en ce que le catalyseur au cuivre est un halogénure de cuivre de métal alcalin.

8. Procédé selon la revendication 7, caractérisé en ce que le catalyseur au cuivre est Li₂CuCl₄.

9. Procédé selon la revendication 2, caractérisé en ce qu'on déprotège le produit couplé par hydrolyse par hydrolyse acide.

10. Procédé selon la revendication 9, caractérisé en ce qu'on effectue l'hydrolyse acide avec des acides organiques et minéraux faibles.

11. Procédé selon la revendication 10, caractérisé en ce qu'on effectue l'hydrolyse acide en ajoutant l'acide formique aqueux au produit couplé.

12. Procédé selon la revendication 3, caractérisé en ce que l'halogénure de benzyle est le chlorure de benzyloxybenzyle.
